# EUROPEAN PATENT SPECIFICATION

(11) **EP 0 653 242 B1**
(45) Date of publication and mention of the grant of the patent: **15.09.1999**
(21) Application number: 94203182.4
(22) Date of filing: 02.11.1994
(51) Int. Cl.: B01J 27/047

(54) **Catalysts, process in which they can be used and process for preparing them**
Katalysatoren, Verfahren unter Verwendung dieser Katalysatoren und Verfahren zur Herstellung dieser Katalysatoren
Catalyseurs, procédés les utilisant et procédés pour les préparer

(30) Priority: 04.11.1993 EP 93402719
(43) Date of publication of application: 17.05.1995
(73) Proprietor: SHELL INTERNATIONALE RESEARCH MAATSCHAPPIJ B.V., 2596 HR Den Haag (NL)
(72) Inventor: Barré, Guy, F-76530 Grand Couronne (FR); Havil, Patrick Frederic Camille, F-76530 Grand Couronne (FR); Lebigre, Sylvie Claude, F-76530 Grand Couronne (FR)

(56) References cited:
- DE-B- 1 165 185
- FR-A- 1 355 210
- FR-A- 2 253 818
- US-A- 3 422 002
- US-A- 4 081 404
- US-A- 5 252 199

## Description

The present invention relates to catalysts containing a refractory oxide carrier and as catalytically active metals a metal of Group VI-B and a metal of Group VIII, to a process comprising contacting a hydrocarbon feedstock containing aromatic compounds in the presence of hydrogen and at elevated temperature and pressure with such catalysts, and to a process for preparing such catalyst.

In US patent specification 3,491,019 a catalyst has been described for use in selective hydrogenation of aromatic hydrocarbons which have more unsaturation than present in a single benzene nucleus. The catalyst comprises a metal of Group VI-B, preferably tungsten, and a metal of Group VIII, preferably nickel, deposited on a silica-alumina composite comprising from about 35 weight percent to about 65 weight percent silica.

In US patent specification 3,422,002 a platinum series metal-molybdena-alumina catalyst is described which has good hydrogenation and hydrodenitrogenation activity.

The present invention relates to catalysts which show improved hydrogenation activity when used in a process in which hydrocarbons containing aromatic compounds are contacted with the catalyst in the presence of hydrogen and at elevated temperature and pressure, more specifically when used in hydrotreating of hydrocarbon fractions containing aromatic compounds and compounds containing sulphur and/or nitrogen. At the moment, such hydrocarbon fractions are usually subjected to a two-stage process in order to reduce the amount of aromatic compounds. In such two-stage process the hydrocarbon fraction is subjected to a first stage in which mainly the amount of sulphur and/or nitrogen containing compounds present in the fraction is brought down, and subsequently to a second stage in which mainly the amount of aromatic compounds is reduced. Such two-stage process is necessary as conventional hydrogenation catalysts for reducing the aromatics content show poor hydrogenation activity in the presence of substantial amounts of sulphur and/or nitrogen containing compounds. The catalysts according to the present invention have a very good hydrogenation activity, even in the presence of relatively large amounts of sulphur and/or nitrogen containing compounds. Further, these catalysts have a good desulphurization and/or denitrogenation activity. This makes that the amount of both aromatic compounds and sulphur and/or nitrogen containing compounds, can be reduced in a single stage process.

The process of the present invention is especially advantageous for hydrotreating gas oil. Gas oils usually contain a relatively large amount of both aromatic compounds and sulphur and/or nitrogen containing compounds. The amount of both kinds of compounds present in the gas oil, must usually be reduced in view of environmental regulations. The process of the present invention can be used for attaining the required reduction in a single stage. With the help of the present process, the requirements for automotive gas oil can be met. It has been found that the catalysts of the present invention are especially active in reducing the amount of mono-aromatics of the final product.

The present invention relates to catalysts containing a refractory oxide carrier which comprises fluorinated alumina, amorphous silica-alumina, siliconaluminophosphate, zeolite and/or clay and as catalytically active metals between 2 and 15% by weight of palladium and between 5 and 40% by weight of tungsten, said weight percentages indicating the amount of metal based on the weight of carrier, in which at least 70% of the total tungsten is present in the form of tungsten sulphide.

Further, the present invention relates to a process for the hydroconversion of hydrocarbons comprising contacting a hydrocarbon feedstock containing aromatic compounds at elevated temperature and pressure and in the presence of hydrogen with a catalyst according to the present invention.

In US patent specification 3,709,814 a cogelled catalyst has been described for use in a combined hydrofining-hydrocracking process. The cogelled catalyst comprises a crystalline zeolitic molecular sieve containing from 0.1 to 2.0 weight percent of palladium, and a gel matrix comprising silica/alumina and nickel or cobalt and further molybdenum or tungsten.

In US patent specification 4,324,645 catalysts have been described for upgrading residual oil fractions by selectively removing CCR while the hydrogen consumption for other functions such as desulphurization and aromatics saturation, is limited. The catalysts comprise a metal function deposited on a porous refractory inorganic support, and have more than about 50% of their pore volume contribution in pores having diameters in the range of about 100 to 200 Å.

The present invention relates to a process in which aromatic compounds are hydrogenated, more specifically to a process in which aromatic compounds are hydrogenated and simultaneously sulphur and/or nitrogen containing compounds are removed. This technical field is fundamentally different from the technical fields to which US patent specifications 3,709,814 and 4,324,645 relate.

The tungsten present on the catalyst of the present invention is mainly present in sulphide form, i.e. at least 70% of the total amount of tungsten present on the catalyst, is present in the form of tungsten sulphide. Usually, more than 95% will be present in the form of tungsten sulphide during normal operation. The catalytic activity of metals in the form of their sulphides differs fundamentally from the catalytic activity of the same metals when present in other forms, such as in the form of oxides or in the elemental form.

In catalyst preparation, the catalysts are usually in the final step subjected to calcination in air, whereby the metals are brought in the form of their oxides. To bring tungsten into the sulphide form, the catalyst can be presulphided prior to contact with the feedstock. Presulphidation can be carried out in many ways, as is known from the prior art.

A typical method for presulphiding the catalyst of the present invention would comprise contacting the catalyst with a sulphiding mixture such as a hydrocarbon oil containing a substantial amount of sulphur-containing compounds at a temperature which is gradually increased from ambient temperature to a temperature of between 150 and 250 °C. The catalyst is to be maintained at this temperature for between 10 and 20 hours. Subsequently, the temperature is to be raised gradually to the operating temperature.

In many cases presulphiding is not necessary. This can be the case when tungsten is converted to tungsten sulphide by contacting the catalyst at process conditions with a feedstock which contains a substantial amount of sulphur containing compounds. Typically, presulphiding is not necessary if the hydrocarbon feedstock comprises at least 0.5% by weight of sulphur containing compounds, said weight percentage indicating the amount of elemental sulphur based on total weight of feedstock. It will be understood that this is advantageous, as presulphiding would take up time during which the reactor cannot be operated.

The catalyst according to the present invention comprises as catalytically active metals between 2 and 15% by weight of palladium and between 5 and 40% by weight of tungsten, both weight percentages indicating the amount of metals based on weight of carrier. It has been found that if the catalytically active metals are present in lower amounts than indicated, the activity of the catalyst becomes too low to be commercially attractive. If, on the other hand, the amount of catalytically active metals is higher, the further increase in catalytic activity does not warrant the costs of the extra amount of metal. This applies especially for palladium. Good results can be obtained with catalysts comprising between 2.5 and 12% by weight of palladium and between 8 and 35% by weight of tungsten. Preferably, between 3 and 10% by weight of palladium and between 10 and 30% by weight of tungsten is present.

It has been found that not only the amount of each of the catalytically active metals influences the activity of the catalyst, but also the molar ratio of palladium on the one hand to tungsten on the other hand. Molar ratios of palladium to tungsten which have been found to give very good hydrogenation activity, are between 0.10 and 1.20, preferably between 0.15 and 1.10. Especially good results have been obtained with the help of a catalyst having a ratio of between 0.20 and 0.50.

The performance of the catalyst will in many cases be further improved by incorporating in the catalyst not only palladium and tungsten , but also cobalt and/or nickel. Without wishing to be bound by any theory, it is thought that the presence of nickel and/or cobalt improves the distribution of the tungsten compound. In order to fully benefit from the presence of the nickel and/or cobalt compound, it is preferred to deposit the nickel and/or cobalt compound on the carrier at the same time as the tungsten compound.

Typical amounts of cobalt and/or nickel compounds -if present at all- have been found to be between 0.5 and 5% by weight, indicating the amount of metal based on the weight of carrier. Preferably, the amount of cobalt and/or nickel is between 1 and 4% by weight.

The catalyst according to the present invention comprises palladium and tungsten, and optionally cobalt and/or nickel. Of the combinations of metals which are possible, a catalyst containing palladium and tungsten and optionally nickel will in most cases give the best results.

It has been found that the carrier on which the catalytically active metals are present, influences the activity of the catalyst.

Further, the catalysts according to the present invention will have a surface area which is such that it gives the catalyst a sufficiently high activity. Typically, the surface area of the catalyst will be at least 80 m²/g, measured according to the BET nitrogen adsorption method. Preferably, the surface area of the catalyst is at least 120 m²/g.

The catalysts of the present invention are typically prepared by incorporating the desired amount of metal(s) into the carrier by means of impregnation or ion-exchange techniques followed by drying and calcining. A preferred method of impregnating the carrier is the so-called pore volume impregnation, which comprises treating a carrier with a volume of impregnating solution which is substantially equal to the pore volume of the carrier. In this way, full use is made of the impregnating solution. Further, it has been found that the catalysts according to the present invention which have been prepared in this way show an especially good performance.

Metal compounds which can be used for preparing the catalysts according to the present invention, are well known in the art. Typical tungsten compounds comprise ammonium metatungstate, sodium tungstate and tungstic acid. The nickel and cobalt compounds which can be used comprise nickel nitrate, nickel chloride, cobalt nitrate and cobalt chloride. Typical palladium compounds for use in an impregnating solution are H₂PdCl₄, palladium nitrate, palladium(II)chloride and its amine complex. The use of H₂PdCl₄ is preferred.

It has been found that catalysts having especially good catalytic activity can be prepared from a precursor made by impregnating a refractory oxide carrier with a solution containing a tungsten compound and optionally a nickel and/or cobalt compound, subsequently further impregnating the carrier with a solution containing a palladium compound, and drying and calcining the thus impregnated carrier at a temperature of between 250 and 400 °C. A precursor process which has been found to give catalysts of improved activity further comprises drying and calcining the carrier which has been impregnated with the solution containing a tungsten compound and optionally a nickel and/or cobalt compound at a temperature of between 400 and 650 °C, and subsequently further impregnating the carrier obtained with a solution containing a palladium compound. As noted hereinbefore presulphidation, to bring the precursor into the catalyst of the invention, can be carried out in many ways, as is known in the art, ex-situ or in-situ provided there is sufficient sulphur content in the feedstock to be treated.

It has been found that a carrier which gives especially good results comprises fluorinated alumina. However, in the impregnation of fluorinated alumina with the appropriate metal salts, the problem of crystallisation of metal oxides is prone to occur. A preparation process in which this problem can be prevented, has been found to comprise impregnating a refractory oxide carrier comprising alumina with a solution containing a tungsten compound and optionally a nickel and/or cobalt compound, drying and calcining the impregnated carrier at a temperature of between 400 and 650 °C, subsequently fluorinating the calcined carrier and drying and calcining the fluorinated carrier at a temperature of between 350 and 550 °C, and subsequently further impregnating the fluorinated carrier with a solution containing a palladium compound, and drying and calcining the thus impregnated carrier at a temperature of between 250 and 400 °C, in which process each calcination is carried out at a lower temperature than the preceding calcination.

The catalysts according to the present invention can be applied in a large range of processes for conversion of hydrocarbon feedstocks containing aromatic compounds, more specifically hydrocarbon feedstocks containing both aromatic compounds and sulphur and/or nitrogen containing compounds. Examples of such processes are hydrocracking, lub oil hydroisomerization and hydrotreating.

If the catalyst is to be applied in a hydrocracking process, the carrier will usually comprise a zeolite and silica and/or alumina as a binder. A carrier which is preferably used comprises zeolite Y and alumina. A hydrocracking process typically comprises contacting a hydrocarbon feedstock boiling between 100 and 500 °C, with a catalyst at a temperature of between 300 and 450 °C and a pressure of between 50 and 150 bar in the presence of hydrogen.

In lub oil hydroisomerization, sulphur and/or nitrogen containing contaminants are removed from a lub oil feedstock, aromatic compounds are hydrogenated and straight chain or slightly branched hydrocarbons are isomerized into further branched hydrocarbons. For application in such lub oil hydroisomerization process, the catalyst according to the present invention will preferably comprise a carrier comprising silica/alumina, siliconaluminophosphate or zeolite with silica and/or alumina as binder. A zeolite which can typically be used consists of silica and alumina and further a small amount of boron. A siliconaluminophosphate which can typically be used is SAPO-11 as described by Union Carbide or SM-3 as described by Chevron. A lub oil process typically comprises contacting a lub oil feedstock at a temperature of between 200 and 450 °C and a pressure up to 100 bar with a catalyst in the presence of hydrogen.

The catalyst has been found to give especially good results when used for hydrotreating of a gas oil. A gas oil is a hydrocarbon fraction comprising at least 70% by weight hydrocarbons boiling between 150 and 450 °C, more specifically between 200 and 450 °C. More specifically, it is advantageous to use the present process in hydrotreating straight run gas oil and light gas oil. With straight run gas oil is meant a gas oil which has not yet been subjected to another conversion process. A typical light gas oil comprises at least 80% by weight of hydrocarbons boiling in the range of between 150 and 450 °C. It has been found that in such process especially good hydrogenation activity is observed for catalysts comprising a carrier containing amorphous silica-alumina or fluorinated alumina.

A hydrotreating process according to the present invention is typically carried out at a temperature of between 200 and 400 °C, preferably between 210 and 350 °C, and a total pressure of between 20 and 200 bar, preferably between 25 and 100 bar. Such process is typically carried out in a set-up as described in European patent applications Nos. 0,553,920 and 0,611,816.

The hydroconversion process according to the present invention is carried out at such process conditions that the product obtained has a boiling point range which is such that at least 2% by weight, preferably at least 5% by weight, of the product obtained has a boiling point between the 90% by weight boiling point of the feedstock and the final boiling point of the feedstock. The hydroconversion process is preferably carried out at such operating conditions that the hydrocarbon product obtained comprises less than 20% by volume of aromatic compounds and less than 0.05% by weight of elemental sulphur, based on total weight of product.

The catalyst according to the present invention will usually slowly deactivate during use in a hydroconversion process. If the activity of the catalyst becomes too low, the catalyst can be regenerated. Methods which can be typically used, have been described in the prior art. In some cases, the catalyst will not be regenerated. In those cases, the catalytically active metals will usually be recovered before disposing of the catalyst. Recovery of the metals can be carried out in the usual way. Such recovery methods have been described in the art.

A typical method for recovering the catalytically active metals comprises removing the deactivated catalyst from the reactor, washing the catalyst to remove the hydrocarbons, burning off the coke, and subsequently recovering the palladium, tungsten and nickel and/or cobalt compounds.

The present invention will hereinafter be further illustrated by some examples.

### EXAMPLES

### Experiment 1

A silica/alumina extrudate of 1.6 mm was prepared from silica/alumina (MS 13-110 ex Grace) which was extruded with the help of a Haake Rheocard 90 and subsequently dried for 16 hours at 120 °C and calcined for 2 hours at 600 °C. The carrier obtained contained 13% by weight of alumina and 87% by weight of silica, and had a surface area of 344 m²/g and a pore volume of 0.77 ml/g. This carrier was impregnated with an aqueous solution containing ammonium metatungstate and a small amount of nickel nitrate, the volume of the solution being about the same as the pore volume of the catalyst carrier (so-called pore volume impregnation). The impregnated carrier was subsequently dried and calcined for 2 hours at 500 °C in air. Subsequently the carrier was impregnated by pore volume impregnation with an aqueous solution containing H₂PdCl₄. The catalyst obtained comprised 0.8% by weight of nickel, 24% by weight of tungsten and 4.3% by weight of palladium, all percentages indicating the amount of metal based on the weight of carrier. This catalyst was calcined for 2 hours at 350 °C under air.

The reactor was loaded with 20 cm³ of catalyst mixed with 80 cm³ of silicon carbide particles of 0.2 mm. The reactor was brought on a total pressure of 50 bar with the help of hydrogen at a gas rate of 500 Nl/kg and a weight hourly space velocity (WHSV) of 1 kg/l.h. The temperature was raised from ambient temperature to 230 °C at a rate of 25 °C/h. The introduction of feed into the reactor was started at 120 °C. The temperature of 230 °C was maintained for 16 hours, after which the temperature was raised further to the operating temperature at a rate of 15 °C/h.

Subsequently the experiment was carried out at a total pressure of 50 bar, a gas rate of 500 Nl/kg, a WHSV of 1 kg/l.h and a reactor temperature which was varied from 330 to 370 °C. Due to the sulphur containing compounds present in the feed, the tungsten present on the catalyst was mainly in the form of tungsten sulphide.

The feed which was used in the experiment was an atmospheric Arabian Light gas oil having the characteristics described in Table 1.

**Table 1**

| | |
|---|---|
| Initial boiling point | 160 °C |
| 10% by weight | 231 °C |
| 50% by weight | 283 °C |
| 90% by weight | 351 °C |
| Final boiling point | 399 °C |
| elemental sulphur | 0.94 %wt |
| elemental nitrogen | 0.0056 %wt |
| benzenes | 74 mmol/100 g feed |
| naphthalenes | 35 mmol/100 g feed |
| phenanthrenes | 5 mmol/100 g feed |
| chrysenes | 1 mmol/100 g feed |
| tetraphenes | 1 mmol/100 g feed |

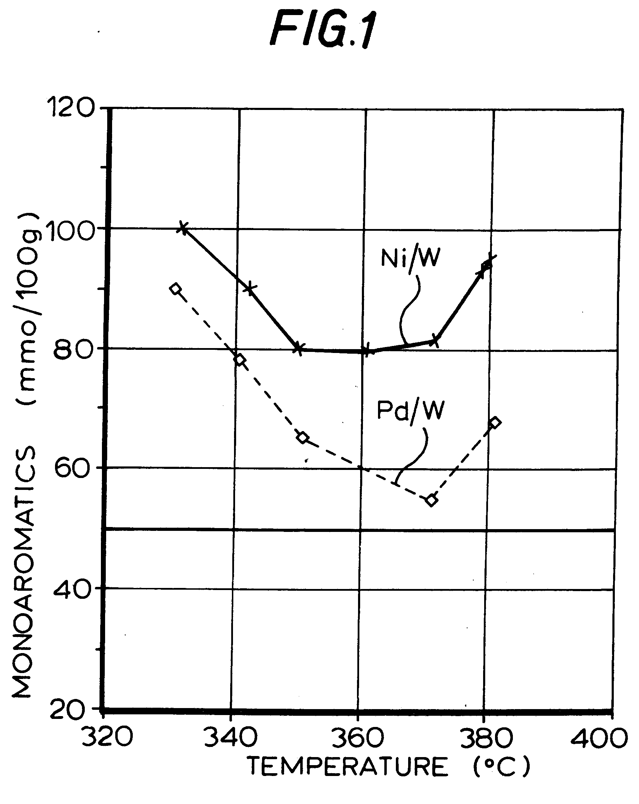

In Figure 1 the amount of monoaromatics in the product is shown. Of the product obtained at an operating temperature of 370 °C, more than 5% by weight of the product had a boiling point between the temperature at which 90% by weight of the feedstock boiled and the final boiling point of the feedstock.

At an operating temperature of 360 °C, the amount of sulphur in the product obtained was 0.0011 %wt.

### Comparative experiment

A silica/alumina extrudate of 1.6 mm was prepared from silica/alumina (MS 13-110 ex Grace) which was extruded with the help of a Haake Rheocord 90 and subsequently dried for 16 hours at 120 °C and calcined for 2 hours at 600 °C. The carrier obtained was impregnated by pore volume impregnation with an aqueous solution of ammonium metatungstate and a small amount of nickel nitrate hexahydrate, followed by drying and calcination for 2 hours at 500 °C in air. Subsequently the carrier was impregnated by pore volume impregnation with an aqueous solution containing nickel nitrate in an ammonia solution. The catalyst obtained comprised 24% by weight of tungsten and 5.1% by weight of nickel, both indicating the amount of metal based on the weight of carrier. This catalyst was calcined for 2 hours at 550 °C under air.

The reactor was loaded with 20 cm³ of catalyst mixed with 80 cm³ of silicon carbide particles of 0.2 mm. The reactor was brought on a total pressure of 50 bar with the help of hydrogen at a gas rate of 500 Nl/kg and a weight hourly space velocity of (WHSV) of 1 kg/l.h. The temperature was raised from ambient temperature to 260 °C at a rate of 25 °C/h. The introduction of feed into the reactor, which feed has been described in Table 1, was started at 120 °C. The temperature of 260 °C was maintained for 16 hours, after which the temperature was raised further to the operating temperature at a rate of 15 °C/h.

The results obtained have been shown in Figure 1. Of the product obtained with the help of the comparative catalysts at an operating temperature of 380 °C, about 4% by weight of the product boiled between the temperature at which 90% by weight of the feedstock boiled and the final boiling point of the feedstock.

At an operating temperature of 360 °C, the amount of sulphur in the product obtained was 0.0014 %wt.

### Experiment 2

Alumina Catapal Al 3900 P from Harshaw was kneaded with acetic acid (1% by weight) and water. The dough was extruded using a Haake Rheocord 90. The extrudates having a diameter of 1.6 mm were dried for 16 hours at 120 °C and subsequently calcined for 2 hours at 600 ° C.

The extrudates obtained were impregnated by pore volume impregnation with an aqueous solution containing ammonium metatungstate and a small amount of nickel nitrate hexahydrate. The partially prepared catalyst was dried for 2 hours at 200 °C and calcined for 2 hours at 500 °C.

Subsequently, the partially prepared catalyst was fluorinated with the help of an aqueous solution of ammonium difluoride followed by drying for 2 hours at 200 °C and calcining for 4 hours at 480 °C.

Finally, the partially prepared catalyst was impregnated by pore volume impregnation with an aqueous solution containing H₂PdCl₄, followed by drying for 2 hours at 200 °C and calcination for 2 hours at 350 °C.

The final catalyst obtained comprised 1.6% by weight of nickel, 24% by weight of tungsten and 4.3% by weight of palladium, all amount of metal on weight of carrier, and 2.9% by weight of fluorine. The surface area of the catalyst obtained was 143 m²/g.

The reactor was loaded with 20 cm³ of catalyst mixed with 100 cm³ of silicon carbide particles of 0.2 mm.

The feedstock which was used has been described in Table 2.

**Table 2**

| | |
|---|---|
| Initial boiling point | 141 °C |
| 10% by weight | 229 °C |
| 50% by weight | 290 °C |
| 90% by weight | 377 °C |
| Final boiling point | 431 °C |
| elemental sulphur | 3.1 %wt |
| elemental nitrogen | 0.070 %wt |
| benzene | 114 mmol/100 g feed |
| naphthalene | 144 mmol/100 g feed |
| phenanthrene | 55 mmol/100 g feed |
| chrysene | 4 mmol/100 g feed |
| tetraphene | 5 mmol/100 g feed |

The process was carried out at a total pressure of 100 bar, a gas rate of 1000 Nl/kg, a weight hourly space velocity of (WHSV) of 1 kg/l.h and a temperature which was gradually increased from 310 to 430 °C. Due to the sulphur containing compounds present in the feed, the tungsten present on the catalyst was mainly in the form of tungsten sulphide.

At an operating temperature of 370 °C, a product was obtained having an initial boiling point of 84 °C and a final boiling point of 431 °C. More than 5% by weight of the product had a boiling point between the 90 %wt boiling point of the feedstock and the final boiling point of the feedstock. Further features of the product have been described in Table 3.

**Table 3**

| | |
|---|---|
| monoaromatics | 128 mmol/100 g feed |
| (of which: benzene | 107 mmol/100 g feed) |
| naphthalene | 4 mmol/100 g feed |
| phenanthrene | 0.4 mmol/100 g feed |
| chrysene | 0.1 mmol/100 g feed |
| tetraphene | 0.4 mmol/100 g feed |

## Claims

1. Catalyst containing a refractory oxide carrier which comprises fluorinated alumina, amorphous silica-alumina, siliconaluminophosphate, zeolite and/or clay and as catalytically active metals between 2 and 15% by weight of palladium, and between 5 and 40% by weight of tungsten, said weight percentages indicating the amount of metal based on the weight of carrier, in which at least 70% of the total tungsten present is in the form of tungsten sulphide.

2. Catalyst according to claim 1, which catalyst further comprises as catalytically active metal between 0.5 and 5% by weight of cobalt and/or nickel, indicating the amount of metal based on the weight of carrier.

3. Catalyst according to claim 1 and/or 2, which catalyst comprises between 3 and 10% by weight of palladium, and further between 10 and 30% by weight of tungsten.

4. Catalyst according to any one of claims 1 to 3, in which catalyst the molar ratio of palladium to tungsten is between 0.10 and 1.20.

5. Catalyst according to any one of claims 1 to 4, in which the catalyst comprises palladium, tungsten, and nickel.

6. Catalyst according to claim 1, in which the refractory oxide carrier comprises amorphous silica-alumina or fluorinated alumina.

7. Catalysts according to any one of claims 1 to 6, in which the catalyst has a surface area of at least 80 m²/g.

8. Process for preparing a catalyst, which process comprises impregnating a refractory oxide carrier with a solution containing a tungsten compound and optionally a nickel and/or cobalt compound, subsequently further impregnating the carrier with a solution containing a palladium compound, and drying and calcining the thus impregnated carrier at a temperature of between 250 and 400 °C, to provide a catalyst precursor which is further treated by conversion of the refractory oxide carrier and by presulphidation as necessary, to give a catalyst as claimed in any one of claims 1 to 7.

9. Process according to claim 8, which process further comprises drying and calcining the carrier which has been impregnated with the solution containing a tungsten compound, and optionally a nickel and/or cobalt compound, at a temperature of between 400 and 650 °C.

10. Process according to claim 9, which process comprises impregnating a refractory oxide carrier comprising alumina with a solution containing a tungsten compound and optionally a nickel and/or cobalt compound, drying and calcining the impregnated carrier at a temperature of between 400 and 650 °C, subsequently fluorinating the calcined carrier and drying and calcining the fluorinated carrier at a temperature of between 350 and 550 °C, and subsequently further impregnating the fluorinated carrier with a solution containing a palladium compound, and drying and calcining the thus impregnated carrier at a temperature of between 250 and 400 °C, in which process each calcination is carried out at a lower temperature than the preceding calcination.

11. Process for hydroconversion of hydrocarbons comprising contacting a hydrocarbon feedstock containing aromatic compounds at elevated temperature and pressure and in the presence of hydrogen with a catalyst according to any one of claims 1 to 7.

12. Process according to claim 11, in which process the hydrocarbon feedstock comprises aromatic compounds and sulphur and/or nitrogen containing compounds.

13. Process according to claim 12, which process is a lub oil hydroisomerization process.

14. Process according to claim 12, which process is a hydrocracking process.

15. Process according to claim 12, which process is a hydrotreating process which is carried out at a temperature of between 200 and 400 °C and a total pressure of between 20 and 200 bar.

16. Process according to claim 15, in which process the feedstock is a gas oil.

17. Process according to any one of claims 12 to 16, in which process the hydrocarbon feedstock comprises at least 0.5% by weight of sulphur containing compounds, indicating the amount of elemental sulphur based on total weight of feedstock.

18. Process according to any one of claims 12 to 17, in which process the hydrocarbon product obtained comprises less than 20% by volume of aromatic compounds and less than 0.05% by weight of elemental sulphur based on total weight of product.

19. Catalyst obtainable by a process according to any one of claims 8 to 10.

## Patentansprüche

1. Katalysator, der einen Träger aus feuerfestem Oxid, das fluoriertes Aluminiumoxid, amorphes Siliziumoxid-Aluminiumoxid, Silicoaluminophosphat, Zeolith und/oder Ton umfaßt, und als katalytisch aktive Metalle 2 bis 15 Gew.-% Palladium und 5 bis 40 Gew.-% Wolfram enthält, wobei diese Gewichtsprozentsätze die Metallmenge, bezogen auf das Gewicht des Trägers, angeben und worin wenigstens 70 % des insgesamt vorliegenden Wolframs in der Form von Wolframsulfid zugegen sind.

2. Katalysator nach Anspruch 1, der zusätzlich als katalytisch aktives Metall 0,5 bis 5 Gew.-% Kobalt und/oder Nickel umfaßt, worin die angegebene Metallmenge auf das Trägergewicht bezogen ist.

3. Katalysator nach Anspruch 1 und/oder 2, welcher Katalysator 3 bis 10 Gew.-% Palladium und weiterhin 10 bis 30 Gew.-% Wolfram umfaßt.

4. Katalysator nach einem der Ansprüche 1 bis 3, worin das Molverhältnis Palladium zu Wolfram 0,10 bis 1,20 beträgt.

5. Katalysator nach einem der Ansprüche 1 bis 4, worin der Katalysator Palladium, Wolfram und Nickel umfaßt.

6. Katalysator nach Anspruch 1, worin der Träger aus feuerfestem Oxid amorphes Siliziumoxid-Aluminiumoxid oder fluoriertes Aluminiumoxid umfaßt.

7. Katalysatoren nach einem der Ansprüche 1 bis 6, worin der Katalysator eine Oberfläche von wenigstens 80 m²/g aufweist.

8. Verfahren zur Herstellung eines Katalysators, welches Verfahren ein Imprägnieren eines Trägers aus feuerfestem Oxid mit einer eine Wolframverbindung und gegebenenfalls eine Nickel- und/oder Kobaltverbindung enthaltenden Lösung, anschließend ein weiteres Imprägnieren des Trägers mit einer eine Palladiumverbindung enthaltenden Lösung und ein Trocknen und Kalzinieren des so imprägnierten Trägers bei einer Temperatur von 250 bis 400°C umfaßt, zur Ausbildung eines Katalysatorvorläufers, der weiter durch Umwandlung des Trägers aus feuerfestem Oxid und durch Präsulfidieren, soferne erforderlich, behandelt wird, um einen Katalysator zu ergeben, wie in einem der Ansprüche 1 bis 7 beansprucht.

9. Verfahren nach Anspruch 8, welches Verfahren weiterhin ein Trocknen und Kalzinieren des Trägers, der mit der eine Wolfram- und gegebenenfalls eine Nickel- und/oder Kobaltverbindung enthaltenden Lösung imprägniert worden ist, bei einer Temperatur von 400 bis 650°C umfaßt.

10. Verfahren nach Anspruch 9, welches Verfahren ein Imprägnieren eines Trägers aus Aluminiumoxid umfassendem feuerfestem Oxid mit einer Lösung, die eine Wolframverbindung und gegebenenfalls eine Nickel- und/oder Kobaltverbindung enthält, ein Trocknen und Kalzinieren des imprägnierten Trägers bei einer Temperatur von 400 bis 650°C, anschließend ein Fluorieren des kalzinierten Trägers und ein Trocknen und Kalzinieren des fluorierten Trägers bei einer Temperatur von 350 bis 550°C und anschließend ein weiteres Imprägnieren des fluorierten Trägers mit einer eine Palladiumverbindung enthaltenden Lösung und ein Trocknen und Kalzinieren des so erhaltenen imprägnierten Trägers bei einer Temperatur von 250 bis 400°C umfaßt, in welchem Verfahren jede Kalzinierung bei einer niedrigeren Temperatur ausgeführt wird als die vorangehende Kalzinierung.

11. Verfahren zur Hydrokonversion von Kohlenwasserstoffen, umfassend ein Inkontaktbringen eines aromatische Verbindungen enthaltenden Kohlenwasserstoffeinsatzmaterials bei erhöhter Temperatur und erhöhtem Druck und in Gegenwart von Wasserstoff mit einem Katalysator nach einem der Ansprüche 1 bis 7.

12. Verfahren nach Anspruch 11, in welchem Verfahren das Kohlenwasserstoffeinsatzmaterial aromatische Verbindungen und Schwefel und/oder Stickstoff enthaltende Verbindungen umfaßt.

13. Verfahren nach Anspruch 12 welches Verfahren ein Schmierölhydroisomerisationsverfahren ist.

14. Verfahren nach Anspruch 12 welches Verfahren ein Hydrocrackverfahren ist.

15. Verfahren nach Anspruch 12 welches Verfahren ein Hydrotreatingverfahren ist, das bei einer Temperatur von 200 bis 400°C und bei einem Gesamtdruck von 20 bis 200 bar ausgeführt wird.

16. Verfahren nach Anspruch 15, in welchem Verfahren das Einsatzmaterial ein Gasöl ist.

17. Verfahren nach einem der Ansprüche 12 bis 16, in welchem Verfahren das Kohlenwasserstoffeinsatzmaterial wenigstens 0,5 Gew.-% schwefelhältige Verbindungen umfaßt, wobei die Menge an elementarem Schwefel auf das Gesamtgewicht des Einsatzmaterials angegeben ist.

18. Verfahren nach einem der Ansprüche 12 bis 17, in welchem Verfahren das erhaltene Kohlenwasserstoffprodukt weniger als 20 Vol.-% aromatische Verbindungen und weniger als 0,05 Gew.-% elementaren Schwefel, bezogen auf das Gesamtgewicht des Produktes, umfaßt.

19. Katalysator, erhältlich nach einem Verfahren gemäß einem der Ansprüche 8 bis 10.

## Revendications

1. Catalyseur contenant un support d'oxyde réfractaire qui comprend de l'alumine fluorée, de la silice-alumine amorphe, du silicoaluminophosphate, de la zéolite et/ou de l'argile et, comme métaux catalytiquement actifs, entre 2 et 15 % en poids de palladium et entre 5 et 40 % en poids de tungstène, lesdits pourcentages en poids indiquant la quantité de métal par rapport au poids de support, dans lequel au moins 70 % du tungstène total sont présents sous la forme de sulfure de tungstène.

2. Catalyseur suivant la revendication 1, lequel catalyseur comprend de plus comme métal catalytiquement actif entre 0,5 et 5 % en poids de cobalt et/ou de nickel, indiquant la quantité de métal par rapport au poids de support.

3. Catalyseur suivant l'une ou l'autre des revendications 1 et/ou 2, lequel catalyseur comprend entre 3 et 10 % en poids de palladium, et de plus entre 10 et 30 % en poids de tungstène.

4. Catalyseur suivant l'une quelconque des revendications 1 à 3, catalyseur dans lequel le rapport molaire du palladium au tungstène se situe entre 0,10 et 1,20.

5. Catalyseur suivant l'une quelconque des revendications 1 à 4, dans lequel ledit catalyseur comprend du palladium, du tungstène et du nickel.

6. Catalyseur suivant la revendication 1, dans lequel le support d'oxyde réfractaire comprend de la silice-alumine amorpne ou de l'alumine fluorée.

7. Catalyseurs suivant l'une quelconque des revendications 1 à 6, dans lesquels le catalyseur a une aire superficielle d'au moins 80 m²/g.

8. Procédé de préparation d'un catalyseur, lequel procédé comprend l'imprégnation d'un support d'oxyde réfractaire avec une solution contenant un composé de tungstène et éventuellement un composé de nickel et/ou de cobalt, ensuite la nouvelle imprégnation du support avec une solution contenant un composé de palladium et le séchage et la calcination du support ainsi imprégné à une température entre 250 et 400°C, pour former un précurseur de catalyseur qui est de plus traité par conversion du support d'oxyde réfractaire et par présulfuration en fonction des nécessités, pour donner un catalyseur suivant l'une quelconque des revendications 1 à 7.

9. Procédé suivant la revendication 8, lequel procédé comprend de plus le séchage et la calcination du support qui a été imprégné avec la solution contenant un composé de tungstène et éventuellement un composé de nickel et/ou de cobalt, à une température entre 400 et 650°C.

10. Procédé suivant la revendication 9, lequel procédé comprend l'imprégnation d'un support d'oxyde réfractaire comprenant de l'alumine avec une solution contenant un composé de tungstène et éventuellement un composé de nickel et/ou de cobalt, le séchage et la calcination du support imprégné à une température entre 400 et 650°C, ensuite la fluoration du support calciné et le séchage et la calcination du support fluoré à une température entre 350 et 550°C, et ensuite à nouveau l'imprégnation du support fluoré avec une solution contenant un composé de palladium et le séchage et la calcination du support ainsi imprégné à une température entre 250 et 400°C, procédé dans lequel chaque calcination est réalisée à une température inférieure à celle de la calcination précédente.

11. Procédé d'hydroconversion d'hydrocarbures comprenant la mise en contact d'une charge d'alimentation hydrocarbonée contenant des composés aromatiques à température et pression élevées et en présence d'hydrogène avec un catalyseur suivant l'une quelconque des revendications 1 à 7.

12. Procédé suivant la revendication 11, procédé dans lequel la charge d'alimentation hydrocarbonée comprend des composés aromatiques et des composés contenant du soufre et/ou de l'azote.

13. Procédé suivant la revendication 12, lequel procédé est un procédé d'hydroisomérisation d'huile lubrifiante.

14. Procédé suivant la revendication 12, lequel procédé est un procédé d'hydrocraquage.

15. Procédé suivant la revendication 12, lequel procédé est un procédé d'hydrotraitement qui est réalisé à une température entre 200 et 400°C et à une pression totale entre 20 et 200 bars.

16. Procédé suivant la revendication 15, procédé dans lequel la charge d'alimentation est un gasoil.

17. Procédé suivant l'une quelconque des revendications 12 à 16, procédé dans lequel la charge d'alimentation hydrocarbonée comprend au moins 0,5 % en poids de composés contenant du soufre, indiquant la quantité de soufre élémentaire par rapport au poids total de la charge d'alimentation.

18. Procédé suivant l'une quelconque des revendications 12 à 17, procédé dans lequel le produit hydrocarboné obtenu comprend moins de 20 % en volume de composés aromatiques et moins de 0,05 % en poids de soufre élémentaire par rapport au poids total de produit.

19. Catalyseur obtenable par un procédé suivant l'une quelconque des revendications 8 à 10.
